# EUROPEAN PATENT SPECIFICATION

(11) **EP 4 262 655 B1**
(45) Date of publication and mention of the grant of the patent: **10.12.2025**
(21) Application number: 21834931.4
(22) Date of filing: 17.12.2021
(51) Int. Cl.: A61F 13/02

(54) **DUAL-SIDED ADHESIVE TAPES WITH ON-DEMAND ADHESION**
DOPPELSEITIGE KLEBEBÄNDER MIT ON-DEMAND HAFTUNG
BANDES ADHÉSIVES DOUBLE FACE AVEC ADHÉSION ON-DEMAND

(30) Priority: 21.12.2020 US 202063128241 P
(43) Date of publication of application: 25.10.2023
(73) Proprietor: Solventum Intellectual Properties Company, Maplewood, MN 55144 (US)
(72) Inventor: GOLD, Anne C. F., Saint Paul, Minnesota 55133-3427 (US); GUTTMANN, Silvia G. B., Saint Paul, Minnesota 55133-3427 (US); SHERMAN, Audrey A., Saint Paul, Minnesota 55133-3427 (US); SHAVER, Jonah, Saint Paul, Minnesota 55133-3427 (US); CORRIGAN, Thomas R., Saint Paul, Minnesota 55133-3427 (US)
(74) Representative: Vossius & Partner Patentanwälte Rechtsanwälte mbB
(86) International application number: PCT/IB2021/061955
(87) International publication number: WO 2022/137062

(56) References cited:
- WO-A1-2020/159675
- US-A1- 2009 104 402
- US-A1- 2012 041 402
- US-A1- 2016 015 570
- US-A1- 2017 014 273

## Description

### Background

US 2009/104402 A1 discloses a taping tape that includes: a base material having elasticity; an adhesive layer formed on one side of the base material; and plural slits that are formed along one direction on an inner side of the base material and are arranged parallel to each other, wherein both side edge portions of the base material in the vicinities of end portions of the slits are formed obliquely to each other towards a longitudinal direction center of the base material from the vicinities of the end portions of the slits to base material end portions positioned on extensions of the slits, and the end portions of the slits curve towards the center of the base material along the side edge portions.

US 2016/015570 A1 discloses a medical dressing comprising: a first major surface, second major surface, opposite the first major surface, and a perimeter, wherein the second major surface comprises an adhesive; a backing layer that is elastic; a support material secured to the backing layer that is less elastic than the backing layer, wherein the support material extends adjacent the entire perimeter but is not continuous across the entire dressing; a plurality of slits through the support material, wherein each slit is a long narrow through cut in the support material and wherein each slit is spaced from an adjacent slit.

WO 2020/159675 A1 discloses a dressing material, comprising: a first layer comprising a first film of non-porous material having a plurality of openings; a second layer adjacent to the first layer, the second layer comprising a manifold having a plurality of slits; and a third layer adjacent to the second layer and comprising a second film of non-porous material having raised features; wherein at least some of the plurality of openings, some of the plurality of slits, and some of the raised features are aligned to define a tear line.

### Summary

Disclosed herein are adhesive articles, medical constructions that include these adhesive articles, and methods of preparing the adhesive articles. The invention is as defined in independent claims 1 and 10. Optional features are found in the dependent claims.

In some embodiments, the adhesive article comprises a layer of adhesive with a first major surface and a second major surface, and a first backing layer with a first major surface and a second major surface, where the first major surface of the first backing layer is in contact with the second major surface of the adhesive layer. The first backing layer comprises a plurality of cuts, wherein the plurality of cuts are gaps but the gaps are not visible to the naked eye when the adhesive article is in an unstressed state, but in a stressed state, at least some of the cuts become gaps that permit adhesion of the second major surface of the adhesive layer to a substrate surface. In some embodiments, the adhesive article further comprises a second backing layer in contact with the first major surface of the adhesive layer.

Also disclosed are adhesive constructions. In some embodiments, the adhesive construction comprises a first substrate surface, and an adhesive article attached to the first substrate surface. The adhesive article is described above and comprises a layer of adhesive with a first major surface and a second major surface, and a first backing layer with a first major surface and the second major surface, where the first major surface of the first backing layer is in contact with the second major surface of the adhesive layer. The first backing layer comprises a plurality of cuts, where the cuts are gaps, but at least some of the gaps are not visible to the naked eye when the adhesive article is in a first state, and at least some of the gaps that are not visible to the naked eye in the first state become gaps when the adhesive article is in a second state, where the second state is a state of higher stress of bending, stretching or a combination thereof. The first major surface of the adhesive layer is in contact with the first substrate surface, and the gaps in the first backing layer permit adhesion of the layer of adhesive to the surface of a second substrate.

Also disclosed are methods of preparing adhesive articles. In some embodiments, the method comprises providing a first backing layer with a first major surface and a second major surface, modifying the first backing layer by cutting a plurality of cuts into the first backing layer without substantially removing material from the first backing layer, such that in an unstressed state the cuts are gaps, but the gaps are not visible to the naked eye but in a stressed state at least some of the gaps become visible to the naked eye, providing an adhesive, and forming a layer of adhesive on the first major surface of the first backing layer such that the adhesive layer has a first major surface and a second major surface and the second major surface of the adhesive layer is in contact with first major surface of the first backing layer.

### Brief Description of the Drawings

The present application may be more completely understood in consideration of the following detailed description of various embodiments of the disclosure in connection with the accompanying drawings.
Figure 1 is a cross sectional view of an article of this disclosure.
Figure 2A is a top view of an article of this disclosure.
Figure 2B is a top view of another article of this disclosure.
Figure 3A shows a top view of the stretch activated article of Figure 2A.
Figure 3B shows a top view of the stretch activated article of Figure 2B.
Figure 4 is a cross sectional view of another article of this disclosure.
Figure 5A is a top view of another article of this disclosure.
Figure 5B shows the stretch activated article of Figure 5A.
Figure 5C shows the stretch de-activated article of Figure 5B.

In the following description of the illustrated embodiments, reference is made to the accompanying drawings, in which is shown by way of illustration, various embodiments in which the disclosure may be practiced. It is to be understood that the embodiments may be utilized and structural changes may be made without departing from the scope of the present disclosure. The figures are not necessarily to scale. Like numbers used in the figures refer to like components. However, it will be understood that the use of a number to refer to a component in a given figure is not intended to limit the component in another figure labeled with the same number.

### Detailed Description

Adhesives have been used for a variety of marking, holding, protecting, sealing and masking purposes. Adhesive tapes generally comprise a backing, or substrate, and an adhesive. One type of adhesive, a pressure sensitive adhesive, is particularly preferred for many applications.

Pressure sensitive adhesives are well known to one of ordinary skill in the art to possess certain properties at room temperature including the following: (1) aggressive and permanent tack, (2) adherence with no more than finger pressure, (3) sufficient ability to hold onto an adherend, and (4) sufficient cohesive strength to be removed cleanly from the adherend. Materials that have been found to function well as pressure sensitive adhesives are polymers designed and formulated to exhibit the requisite viscoelastic properties resulting in a desired balance of tack, peel adhesion, and shear strength. The most commonly used polymers for preparation of pressure sensitive adhesives are natural rubber, synthetic rubbers (e.g., styrene/butadiene copolymers (SBR) and styrene/isoprene/styrene (SIS) block copolymers), various (meth)acrylate (e.g., acrylate and methacrylate) copolymers and silicones. Each of these classes of materials has advantages and disadvantages.

In many applications it is desirable to have a dual-sided tape. Dual-sided tapes, also called "transfer tapes" are adhesive tapes that have adhesive on both exposed surfaces. In some transfer tapes, the exposed surfaces are simply the two surfaces of a single adhesive layer. Other transfer tapes are multi-layer transfer tapes with at least two adhesive layers that may be the same or different, and in some instances intervening layers that may not be adhesive layers.

One difficulty with dual-sided tapes is that it can be difficult to transport, handle, and apply to a substrate because the tapes have 2 tacky adhesive layers exposed. The use of release liners covering one or both of the adhesive layers can make the tapes easier to transport, handle, and apply to a substrate. However, the use of release liners can be expensive and inconvenient as the release liners need to be removed to expose the adhesive layer, and the liner discarded. In this disclosure, tapes are described that have two adhesive surfaces where the second adhesive surface is in contact with a backing layer. The backing layer comprises a plurality of cuts, where the plurality of cuts are gaps but the gaps are not visible to the naked eye when the adhesive article is in an unstressed state, but in a stressed state, at least some of the cuts become gaps that permit adhesion of the first major surface of the adhesive layer to a substrate surface. In this way, the second adhesive surface is an adhesive-on-demand surface, where the second adhesive surface is exposed upon application of a stress to the adhesive article.

Also disclosed herein are tape articles where both adhesive surfaces are in contact with a backing layer, where both backing layers comprise a plurality of cuts as described above. In these articles, the adhesive articles are non-adhesive until stress is applied to the adhesive article.

The term "adhesive" as used herein refers to polymeric compositions useful to adhere together two adherends. Examples of adhesives are pressure sensitive adhesives and gel adhesives.

Pressure sensitive adhesive compositions are well known to those of ordinary skill in the art to possess properties including the following: (1) aggressive and permanent tack, (2) adherence with no more than finger pressure, (3) sufficient ability to hold onto an adherend, and (4) sufficient cohesive strength to be cleanly removable from the adherend. Materials that have been found to function well as pressure sensitive adhesives are polymers designed and formulated to exhibit the requisite viscoelastic properties resulting in a desired balance of tack, peel adhesion, and shear holding power. Obtaining the proper balance of properties is not a simple process.

As used herein, the term "gel adhesive" refers to a tacky semi-solid crosslinked matrix containing a liquid or a fluid that is capable of adhering to one or more substrates. The gel adhesives may have some properties in common with pressure sensitive adhesives, but they are not pressure sensitive adhesives. "Hydrogel adhesives" are gel adhesives that have water as the fluid contained within the crosslinked matrix.

The term "cut" as used herein refers to a narrow opening made in a substrate by the penetration of a cutting tool such that substantially no material is removed from the substrate by the process of cutting. The terms "cuts" and "slits" are used interchangeably.

The term "(meth)acrylate" refers to monomeric acrylic or methacrylic esters of alcohols. Acrylate and methacrylate monomers or oligomers are referred to collectively herein as "(meth)acrylates". Materials referred to as "(meth)acrylate-based" are materials that contain one or more (meth)acrylate materials and may optionally include additional co-polymerizable materials.

The terms "siloxane-based" as used herein refer to polymers or units of polymers that contain siloxane units. The terms silicone or siloxane are used interchangeably and refer to units with dialkyl or diaryl siloxane (-SiR₂O-) repeating units.

The terms "room temperature" and "ambient temperature" are used interchangeably to mean temperatures in the range of 20°C to 25°C.

The terms "Tg" and "glass transition temperature" are used interchangeably. If measured, Tg values are determined by Differential Scanning Calorimetry (DSC) at a scan rate of 10°C/minute, unless otherwise indicated. Typically, Tg values for copolymers are not measured but are calculated using the well-known Fox Equation, using the homopolymer Tg values provided by the monomer supplier, as is understood by one of skill in the art.

The term "adjacent" as used herein when referring to two layers means that the two layers are in proximity with one another with no intervening open space between them. They may be in direct contact with one another (e.g. laminated together) or there may be intervening layers.

The term "pattern" as used herein refers to a plurality of cuts, where the plurality of cuts forms an array, and the array is in a pattern, where the pattern may be a "random pattern", meaning that there is no readily discernible repeating unit in the array, or the array may be aligned along at least one axis. In some embodiments, the array is aligned along one axis, in other embodiments, the array is aligned along more than one axis.

The terms "polymer" and "macromolecule" are used herein consistent with their common usage in chemistry. Polymers and macromolecules are composed of many repeated subunits. As used herein, the term "macromolecule" is used to describe a group attached to a monomer that has multiple repeating units. The term "polymer" is used to describe the resultant material formed from a polymerization reaction.

The term "alkyl" refers to a monovalent group that is a radical of an alkane, which is a saturated hydrocarbon. The alkyl can be linear, branched, cyclic, or combinations thereof and typically has 1 to 20 carbon atoms. In some embodiments, the alkyl group contains 1 to 18, 1 to 12, 1 to 10, 1 to 8, 1 to 6, or 1 to 4 carbon atoms. Examples of alkyl groups include, but are not limited to, methyl, ethyl, n-propyl, isopropyl, n-butyl, isobutyl, tert-butyl, n-pentyl, n-hexyl, cyclohexyl, n-heptyl, n-octyl, and ethylhexyl.

The term "aryl" refers to a monovalent group that is aromatic and carbocyclic. The aryl can have one to five rings that are connected to or fused to the aromatic ring. The other ring structures can be aromatic, non-aromatic, or combinations thereof. Examples of aryl groups include, but are not limited to, phenyl, biphenyl, terphenyl, anthryl, naphthyl, acenaphthyl, anthraquinonyl, phenanthryl, anthracenyl, pyrenyl, perylenyl, and fluorenyl.

The term "alkylene" refers to a divalent group that is a radical of an alkane. The alkylene can be straight-chained, branched, cyclic, or combinations thereof. The alkylene often has 1 to 20 carbon atoms. In some embodiments, the alkylene contains 1 to 18, 1 to 12, 1 to 10, 1 to 8, 1 to 6, or 1 to 4 carbon atoms. The radical centers of the alkylene can be on the same carbon atom (i.e., an alkylidene) or on different carbon atoms.

The terms "free radically polymerizable" and "ethylenically unsaturated" are used interchangeably and refer to a reactive group which contains a carbon-carbon double bond which is able to be polymerized via a free radical polymerization mechanism.

The term "gap" as used herein refers to a void space in a substrate that passes through the entire thickness of the substrate. Gaps can be prepared by cutting, slitting, boring, etc. In the current disclosure, gaps are described as not visible to the naked eye when the substrate is in an unstressed state (meaning that one cannot see through the slit backing to see the underlying layer), and the gaps are described as being visible to the naked eye and as forming "apertures" when in a stressed state. The term "aperture" as used herein is used according to the typical definition and is a space through which light passes (meaning that one can see through the slit backing to see the underlying layer). The term "hole" as used herein refers to a void space in the surface of a substrate that is visible to the naked eye in an unstressed state and a stressed state, being an aperture under both an unstressed state and a stressed state.

Disclosed herein are adhesive articles. In some embodiments, the adhesive article comprises a layer of adhesive with a first major surface and a second major surface, and a first backing layer with a first major surface and a second major surface. The first major surface of the first backing layer is in contact with the second major surface of the adhesive layer. The first backing layer comprises a plurality of cuts, where the plurality of cuts are gaps but the gaps are not visible to the naked eye when the adhesive article is in an unstressed state, but in a stressed state, at least some of the cuts become gaps that permit adhesion of the second major surface of the adhesive layer to a substrate surface. In this way, the second major surface of the adhesive layer is an adhesion-on-demand layer.

A wide array of adhesives is suitable for use in the adhesive layer of the adhesive articles of this disclosure. The adhesive layer may be a continuous layer, a discontinuous layer, or may comprises multiple layers or partial layers of adhesives. In some embodiments, the adhesive comprises a pressure sensitive adhesive, in other embodiments the adhesive comprises a gel adhesive. Suitable pressure sensitive adhesives include (meth)acrylate-based pressure sensitive adhesives and siloxane-based pressure sensitive adhesives.

One suitable class of (meth)acrylate-based pressure sensitive adhesives include copolymers derived from: (A) at least one monoethylenically unsaturated alkyl (meth) acrylate monomer (i.e., alkyl acrylate and alkyl methacrylate monomer); and (B) at least one monoethylenically unsaturated free-radically copolymerizable reinforcing monomer. The reinforcing monomer has a homopolymer glass transition temperature (Tg) higher than that of the alkyl (meth)acrylate monomer and is one that increases the glass transition temperature and cohesive strength of the resultant copolymer. Herein, "copolymer" refers to polymers containing two or more different monomers, including terpolymers, tetrapolymers, etc.

Monomer A, which is a monoethylenically unsaturated alkyl acrylate or methacrylate (i.e., (meth)acrylic acid ester), contributes to the flexibility and tack of the copolymer. Generally, monomer A has a homopolymer Tg of no greater than about 0°C. Typically, the alkyl group of the (meth)acrylate has an average of about 4 to about 20 carbon atoms, or an average of about 4 to about 14 carbon atoms. The alkyl group can optionally contain oxygen atoms in the chain thereby forming ethers or alkoxy ethers, for example. Examples of monomer A include, but are not limited to, 2-methylbutyl acrylate, isooctyl acrylate, lauryl acrylate, 4- methyl-2-pentyl acrylate, isoamyl acrylate, sec-butyl acrylate, n-butyl acrylate, n-hexyl acrylate, 2-ethylhexyl acrylate, n-octyl acrylate, n-decyl acrylate, isodecyl acrylate, isodecyl methacrylate, and isononyl acrylate. Other examples include, but are not limited to, poly-ethoxylated or -propoxylated methoxy (meth)acrylates such as acrylates of CARBOWAX (commercially available from Union Carbide) and NK ester AM90G (commercially available from Shin Nakamura Chemical, Ltd., Japan). Suitable monoethylenically unsaturated (meth)acrylates that can be used as monomer A include isooctyl acrylate, 2-ethyl-hexyl acrylate, and n- butyl acrylate. Combinations of various monomers categorized as an A monomer can be used to make the copolymer.

Monomer B, which is a monoethylenically unsaturated free- radically copolymerizable reinforcing monomer, increases the glass transition temperature and cohesive strength of the copolymer. Generally, monomer B has a homopolymer Tg of at least about 10°C. Typically, monomer B is a reinforcing (meth)acrylic monomer, including an acrylic acid, a methacrylic acid, an acrylamide, or a (meth)acrylate. Examples of monomer B include, but are not limited to, acrylamides, such as acrylamide, methacrylamide, N-methyl acrylamide, N-ethyl acrylamide, N- hydroxyethyl acrylamide, diacetone acrylamide, N,N-dimethyl acrylamide, N, N-diethyl acrylamide, N-ethyl-N-aminoethyl acrylamide, N-ethyl-N- hydroxyethyl acrylamide, N,N-dihydroxyethyl acrylamide, t-butyl acrylamide, N,N-dimethylaminoethyl acrylamide, and N-octyl acrylamide. Other examples of monomer B include itaconic acid, crotonic acid, maleic acid, fumaric acid, 2,2-(diethoxy)ethyl acrylate, 2-hydroxyethyl acrylate or methacrylate, 3-hydroxypropyl acrylate or methacrylate, methyl methacrylate, isobornyl acrylate, 2-(phenoxy)ethyl acrylate or methacrylate, biphenylyl acrylate, t-butylphenyl acrylate, cyclohexyl acrylate, dimethyladamantyl acrylate, 2-naphthyl acrylate, phenyl acrylate, N-vinyl formamide, N-vinyl acetamide, N-vinyl pyrrolidone, and N-vinyl caprolactam. Particularly suitable reinforcing acrylic monomers that can be used as monomer B include acrylic acid and acrylamide. Combinations of various reinforcing monoethylenically unsaturated monomers categorized as a B monomer can be used to make the copolymer.

Generally, the (meth)acrylate copolymer is formulated to have a resultant Tg of less than about 0°C and more typically, less than about -10°C. Such (meth)acrylate copolymers generally include about 60 parts to about 98 parts per hundred of at least one monomer A and about 2 parts to about 40 parts per hundred of at least one monomer B. In some embodiments, the (meth)acrylate copolymers have about 85 parts to about 98 parts per hundred or at least one monomer A and about 2 parts to about 15 parts of at least one monomer B.

Examples of suitable (meth)acrylate-based pressure sensitive adhesives that can be applied to skin are described in U.S. Patent No. RE 24,906. In some embodiments, a 97:3 iso-octyl acrylate:acrylamide copolymer adhesive can be used or a 70:15:15 isooctyl acrylate: ethyleneoxide acrylate:acrylic acid terpolymer, as described in US Patent No. 4,737,410. Other useful adhesives are described in US Patent Nos. 3,389,827, 4,112,213, 4,310,509, and 4,323,557.

Another class of suitable pressure sensitive adhesive is siloxane-based adhesives. Siloxane-based pressure sensitive adhesives include, for example, those described in US Patent Nos. 5,527,578 and 5,858,545; and PCT Publication No. WO 00/02966. Specific examples include polydiorganosiloxane polyurea copolymers and blends thereof, such as those described in U.S. Patent No. 6,007,914, and polysiloxane-polyalkylene block copolymers. Other examples of siloxane pressure sensitive adhesives include those formed from silanols, silicone hydrides, siloxanes, epoxides, and (meth)acrylates. When the siloxane pressure sensitive adhesive is prepared from (meth)acrylate-functional siloxanes, the adhesive is sometimes referred to as a siloxane (meth)acrylate.

The siloxane-based adhesive compositions comprise at least one siloxane elastomeric polymer and may contain other components such as tackifying resins. The elastomeric polymers include for example, urea-based siloxane copolymers, oxamide-based siloxane copolymers, amide-based siloxane copolymers, urethane-based siloxane copolymers, and mixtures thereof.

Useful siloxane polyurea block copolymers are disclosed in, e.g., U.S. Patent Nos. 5,512,650, 5,214,119, 5,461,134, and 7,153,924 and PCT Publication Nos. WO 96/35458, WO 98/17726, WO 96/34028, WO 96/34030 and WO 97/40103.

Another useful class of siloxane elastomeric polymer are oxamide-based polymers such as polydiorganosiloxane polyoxamide block copolymers. Examples of polydiorganosiloxane polyoxamide block copolymers are presented, for example, in US Patent Publication No. 2007-0148475.

Another useful class of siloxane elastomeric polymer is amide-based siloxane polymers. Such polymers are similar to the urea-based polymers, containing amide linkages (-N(D)-C(O)-) instead of urea linkages (-N(D)-C(O)-N(D)-), where C(O) represents a carbonyl group and D is a hydrogen or alkyl group.

Another useful class of siloxane elastomeric polymer is urethane-based siloxane polymers such as siloxane polyurea-urethane block copolymers. Siloxane polyurea-urethane block copolymers include the reaction product of a polydiorganosiloxane diamine (also referred to as siloxane diamine), a diisocyanate, and an organic polyol. Such materials are structurally very similar to the structure of Formula I except that the - N(D)-B-N(D)- links are replaced by -O-B-O- links. Examples are such polymers are presented, for example, in US Patent No. 5,214,119.

In some embodiments, the siloxane-based pressure sensitive adhesive further comprises a siloxane tackifying resin. Siloxane tackifying resins have in the past been referred to as "silicate" tackifying resins, but that nomenclature has been replaced with the term "siloxane tackifying resin". The siloxane tackifying resins are added in sufficient quantity to achieve the desired tackiness and level of adhesion. In some embodiments, a plurality of siloxane tackifying resins can be used to achieve desired performance.

Suitable siloxane tackifying resins are commercially available from sources such as Dow Corning (e.g., DC 2-7066), Momentive Performance Materials (e.g., SR545 and SR1000), and Wacker Chemie AG (e.g., BELSIL TMS-803).

Another class of adhesives used in medical applications are silicone gels. As used herein, the terms "siloxane" and "silicone" are used interchangeably. The term siloxane is replacing silicone in common usage, but both terms are used in the art. Silicone gel (crosslinked poly dimethylsiloxane ("PDMS")) materials have been used for dielectric fillers, vibration dampers, and medical therapies for promoting scar tissue healing. Lightly crosslinked silicone gels are soft, tacky, elastic materials that comprise relatively high levels of fluids (liquids). Silicone gels are typically softer than silicone pressure sensitive adhesives, resulting in less discomfort when adhered to skin. The combination of reasonable adhesive holding power on skin and low skin trauma upon removal, make silicone gels suitable for gentle to skin adhesive applications.

Silicone gel adhesives provide good adhesion to skin with gentle removal force and have the ability to repositioned. Examples of commercially available silicone gel adhesive systems include products marketed with the trade names: Dow Corning MG 7-9850, WACKER 2130, BLUESTAR 4317 and 4320, and NUSIL 6345 and 6350. These gentle to the skin adhesives are formed by an addition cure reaction between vinyl-terminated PDMS and hydrogen terminated PDMS, in the presence of a hydrosilylation catalyst (e.g., platinum complex). Vinyl-terminated and hydrogen terminated PDMS chains are referred to as 'functionalized' silicones due to their specific chemical moieties. Individually, such functional silicones are generally not reactive; however, together they form a reactive silicone system. Additionally, silicone resins (tackifiers sometimes referred to as "silicate resins") and PDMS with multiple hydrogen functionalities (crosslinkers) can be formulated to modify the adhesive properties of the gel.

In some embodiments, the adhesive layer comprises a multi-layer adhesive. The multi-layer adhesive comprises at least two adhesive layers, where the first adhesive layer comprises the first major surface of the adhesive layer of the adhesive article, and the second adhesive layer comprises the second major surface of the adhesive layer of the adhesive article. The first and second adhesive layers may be the same or different. In this way, the adhesive properties of the first major surface and the second major surface of the can be different.

The first backing layer is typically a thin-film material. The film material is rigid enough to provide support to the adhesive article. The backing layer is modified by cutting to make it flexible enough that it is conformable to anatomical surfaces. As such, when applied to an anatomical surface, it conforms to the surface even when the surface is moved and can stretch and retract. The modification process of the current disclosure permits a wide range of material films to be used that otherwise would not be conformable to anatomical surfaces. Among the classes of materials suitable are thermoplastics, elastomers, and nonwovens. The backing layer may be prepared from a wide range of materials including polyolefins, polyurethanes, polyester, polyether block amides, or natural fiber-based materials. One particularly suitable substrate is the SONTARA nonwoven material from Sontara America, which is a tightly enmeshed nonwoven film with no holes suitable for use in medical barrier and drape applications.

The substrate layer has a wide range of thicknesses. In some embodiments, the thickness is at least 10 micrometers, up to 254 micrometers (10 mils), and in some embodiments the thickness will be from 25 micrometers (1 mil) up to 178 micrometers (7 mils) thick. A wide range of intermediate thicknesses are also suitable.

As mentioned above, the substrate layer contains a plurality of cuts arrayed in a pattern, in some embodiments the pattern may be random, in other embodiments, the pattern is arrayed along at least one axis. The two-dimensional surface of the substrate layer can be viewed as having two primary orthogonal axes, frequently referred to as an x axis and a y axis. Because of how films are made, often the x axis is described as the "Machine Direction" or MD and the orthogonal y axis is described as the "Cross Direction" or CD. Typically, the plurality of cuts is arrayed in a pattern along the Machine Direction. By this it is meant that the lengths of the plurality of cuts are aligned along the Machine Direction of the substrate layer.

The plurality of cuts arrayed in a pattern are gaps, but these gaps are essentially not visible to the naked eye when the substrate layer is in an unstressed state. At least some of the gaps become visible to the naked eye when the adhesive article is in a stressed state. As used herein the term "stressed state" comprises stretching, bending or a combination thereof. Because the cuts are very thin (i.e. have essentially no width) in the unstressed state, they are typically described by their length and a depth. Generally, the cut extends through the full thickness of the substrate layer, so the depth is the same as the thickness of the substrate layer. One parameter that can be descriptive of the relationship of the length to the width of the cut is the aspect ratio. The term "aspect ratio" is typically used to describe particles, but as used herein it is used to describe hole size or void regions where material is not present. In some embodiments, the aspect ratio (the ratio of length to width) for the cuts is greater than 1000. Upon application of a stress, the aspect ratio for the cuts decreases. An example of a suitable cut is one that is 1 centimeter long and 5 micrometers wide.

In some embodiments, the plurality of cuts may include at least some cuts that are continuous. By continuous it is meant that the cut extends from edge to edge of the surface. These cuts, upon the application of stress, separate to form gaps in the backing that extend from edge to edge and therefore effectively bisect the backing layer.

The cuts may be more complex than the simple linear shape described above. The cuts may have a variety of two-dimensional shapes such as crosses, asterisks, chevrons, letters, numbers, and the like as long as the shape is not visible to the naked eye in the unstressed state and at least some of the cuts become gaps that are visible to the naked eye when the adhesive article is in a stressed state. Additionally, not only do the gaps become visible to the naked eye upon application of stress to the article, but also the gaps become apertures that light can pass through. In this way, upon application of stress, the formed apertures allow access of the adhesive layer through the substrate layer.

In some embodiments, the cuts are arrayed in a pattern along the Machine Direction of the substrate. Often the cuts are formed by a process known as "skip slitting". In this process, a series of discontinuous slits are formed in the substrate in a linear manner. If one were to follow a line of slits along the substrate surface one would encounter a slit, the slit would end and there would be a region that is not slit, then a new slit would be encountered, that slit would end, a region that is not slit would be encountered, and so forth. This type of pattern is described in PCT Publication No. WO 19/043621. In these patterns, the region between the end of one slit and the start of the next slit is called a bridging region. While a wide array of patterns is suitable, in some embodiments, the patterns have a 50% offset. By this it is meant that when two linear arrays are present in the Machine Direction, when observed in the Cross Direction, the bridging region of the first array corresponds to slits in the second array and vice versa. Additional examples of skip slit patterns are described in US Patent Application No. 62/952789.

In some embodiments, the plurality of cuts is arrayed in a pattern along more than one axis. By this it is meant that at least some of cuts have lengths that are not aligned with the Machine Direction of the substrate layer but are offset from alignment by an angle of up to 90°. Lengths that are offset from alignment with the MD of the substrate layer by 90° are aligned in the Cross Direction.

The plurality of cuts can be made in a number of different ways as long as the method does not involve removing substantial amounts of material from the substrate layer and the cuts form gaps that are essentially not visible to the naked eye in the unstressed state and at least some of gaps become visible to the naked eye when the adhesive article is in a stressed state. Among the methods are those in which the cuts are introduced into the substrate layer when the layer is formed for example by extrusion, molding, machining and the like. Other methods are those in which the cuts are introduced into the substrate layer after the substrate layer is formed such as by cutting using a cutting tool such as a knife, a linear blade, a rotary die blade, a water jet, or a laser beam, or by stamping using a stamping tool. In some embodiments, the cuts are made by feeding the substrate layer into a nip containing a rotary die blade and an anvil such that the die cuts through the substrate layer to form the cut pattern.

Conceptually a slit is a cut through material with minimal thickness. In this way, slits are different from perforations where perforations are permanent gaps in the backing layer. In practice, forming techniques may involve the removal of material, or the formation of a gap between the edges of a slit. Most cutting technologies produce a "kerf", or some physical width to the cut. For example, a laser cutter can ablate some material to create a slit, a router can cut away material to create the slit, even crush cutting can create some deformation on the edges of the material that forms a physical gap. Molding techniques require material between opposing faces of the slit, creating a gap or kerf at the slit. In most cases, the gap or kerf of the slit will be less than the thickness of the material. For example, a slit pattern cut into paper that is .007 inch (178 micrometers) thick might have slits with a gap that is approximately .007 inch (178 micrometers) or less. However, it is understood that the physical gap of the slit could be increased to a factor that is many times larger than the thickness of the substrate. As mentioned above, the slits are formed in the backing layer in such a way as to minimize the removal of material. In this way, the slits are not visible when the article is in an unstressed state, and thus do not form permanent openings in the backing.

In some embodiments, the adhesive articles not only can be activated on demand to permit adhesion, but also can be de-activated on demand to aid in decrease adhesion. In these embodiments, the article comprising slits is stressed in one direction to activate the article to permit adhesion, and when the article is to be removed the article is stressed in an orthogonal direction to de-activate. De-activation causes a shrinking of the gaps in the backing layer and can also cause wrinkling or buckling of the backing layer to aid in the release of the adhesive article.

In some embodiments, the adhesive article further comprises a second backing layer with a first major surface and a second major surface, where the second major surface of the second backing layer is in contact with the first major surface of the adhesive layer. The second backing layer comprises a plurality of cuts, where the plurality of cuts are gaps but the gaps are not visible to the naked eye when the adhesive article is in an unstressed state, but in a stressed state, at least some of the cuts become gaps that permit adhesion of the layer of adhesive to a substrate surface. Examples of suitable second backing layers are described above and include the backing layers described as the first backing layer. The second backing layer may be the same as the first backing layer or it may be different.

An advantage of including a second backing layer in the dual-sided adhesive article, is that both external surfaces are non-adhesive in the unstressed state, permitting easy transportation and handling. In many adhesive articles, a release liner is used to protect the adhesive surface until the article is to be used. In these articles, the release liner needs to be removed when the article is to be used. This can be inconvenient and requires that the removed release liner needs to be discarded or recycled. In the articles of this disclosure that comprise two backing layers, the articles are non-adhesive in the unstressed state and upon the application of a stress, the article becomes an adhesive article. As described above, stress comprises stretching, bending or a combination thereof. In this way the articles can be viewed as an adhesive-on-demand article.

Also disclosed are adhesive constructions. In some embodiments, the adhesive construction comprises a first substrate surface, and an adhesive article attached to the first substrate surface. The adhesive article comprises an adhesive article as described above. In some embodiments, the adhesive article comprises a layer of adhesive with a first major surface and a second major surface, and a first backing layer with a first major surface and the second major surface, where the first major surface of the first backing layer is in contact with the second major surface of the adhesive layer. The first backing layer comprises a plurality of cuts, and wherein the cuts are gaps, but at least some of the gaps are not visible to the naked eye when the adhesive article is in a first state, and at least some of the gaps that are not visible to the naked eye in the first state become gaps when the adhesive article is in a second state. The second state is a state of higher stress of bending, stretching or a combination thereof. The first major surface of adhesive layer is in contact with the first substrate surface, and the gaps in the first backing layer permit adhesion of the second major surface of the layer of adhesive to the surface of a second substrate. In some embodiments, the adhesive article is a medical article and the first substrate surface is mammalian skin.

The adhesive articles have been described in detail above, and may comprise a single or multi-layer adhesive, and may further comprise a second backing layer. Suitable adhesives and backing layers are described above.

A wide array of second substrates can be adhered to the second surface of the adhesive layer when the adhesive article is in a stressed state and the slits in the first backing layer are gaps. In some embodiments, the second substrate comprises an article. Examples of suitable articles are devices. A wide array of devices is suitable including, for example, a wearable medical device, a sensor, a catheter, a vacuum port, a controller for an insulin pump, a monitor, and the like.

Also disclosed herein are methods of preparing adhesive articles. In some embodiments, the method comprises providing a first backing layer with a first major surface and a second major surface, modifying the first backing layer by cutting a plurality of cuts into the substrate layer, such that in an unstressed state the cuts are gaps that are not visible to the naked eye and in a stressed state at least some of the gaps become visible to the naked eye, providing an adhesive, and forming a layer of adhesive on the first major surface of the substrate layer. The adhesive layer has a first major surface and a second major surface and the second major surface of the adhesive layer is in contact with first major surface of the first backing layer.

A wide variety of techniques are suitable for modifying the first backing layer, as long as no material is removed from the first backing layer. In some embodiments, modifying the first backing layer comprises cutting a plurality of cuts into the first backing layer with a knife, a blade, a water jet, or a laser beam. A wide variety of techniques are suitable for modifying the first backing layer. Examples of modification include cutting using a cutting tool such as a knife, a linear blade, a rotary die blade, a water jet, or a laser beam, or by stamping using a stamping tool. In some embodiments, the cuts are made by feeding the first backing layer into a nip containing a rotary die blade and an anvil such that the die cuts through the first backing layer to form the cut pattern.

In many embodiments, the first backing layer is modified prior to forming a layer of adhesive on the first major surface of the substrate layer. In this way, the first backing layer can be modified in a different location or at a different time than the formation of the adhesive article. In some embodiments, the layer of adhesive on the first major surface of the substrate layer is formed prior to modifying the substrate layer. In this wasy, a pre-made tape article can be modified to make it an on-demand dual-sided adhesive article.

As described above, the adhesive layer may be continuous or discontinuous, and may be a multi-layer adhesive. Suitable adhesives are described above.

In some embodiments, the method further comprises providing a second backing layer with a first major surface and a second major surface and contacting the second major surface of the second backing layer to the first major surface of the adhesive layer. The second backing layer, like the first backing layer comprises a plurality of cuts, where the cuts are gaps, but the gaps are not visible to the naked eye but in a stressed state at least some of the gaps become visible to the naked eye.

Articles of this disclosure may be further understood by reference to the figures.

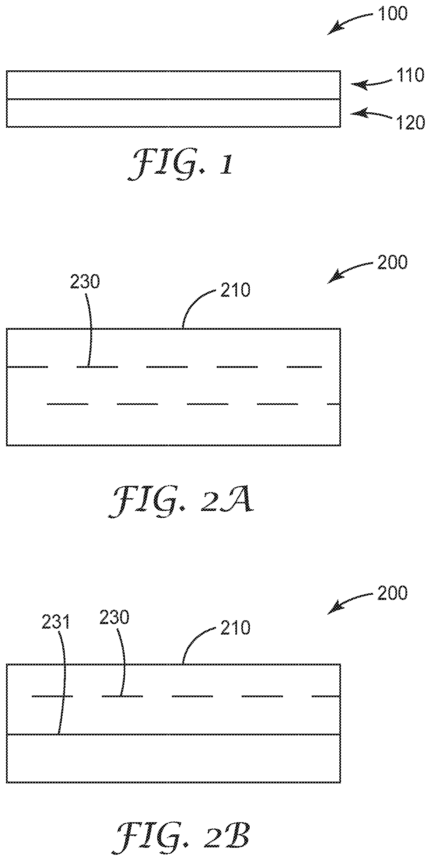

Figure 1 shows a cross-sectional view of article 100 with backing layer 110 and adhesive layer 120. Suitable backing layers and adhesive layers are described above. Backing layer 110 has been modified with slits as described above.

Figures 2A and 2B show top views of embodiments of the articles shown in Figure 1. In the embodiment shown in Figure 2A. the article 200 has backing layer 210 with an array of slits 230. In the embodiment shown in Figure 2B, backing layer 210 with an array of slits 230 and continuous slit 231.

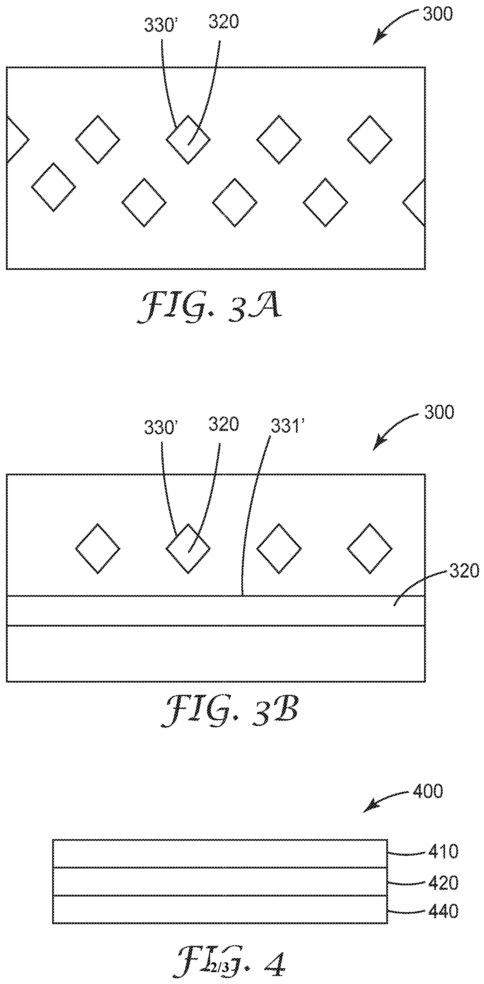

Figures 3A and 3B show top views of articles 300 which are the articles 200 shown in Figures 2A and 2B that have been activated by stretching. In Figure 3A, activated slits 330' form gaps that permit adhesive layer 320 to be exposed. In Figure 3B, as in Figure 3A, activated slits 330' form gaps that permit adhesive layer 320 to be exposed. Additionally, activated slit 331' forms a gap that permits the underlying adhesive layer to be exposed. In Figure 3B the gap 331' is a separation in the backing layer.

Figure 4 shows a cross-sectional view of article 400 which comprises a first backing layer 410, adhesive layer 420, and second backing layer 440. Suitable first and second backing layers and suitable adhesive layers are described above.

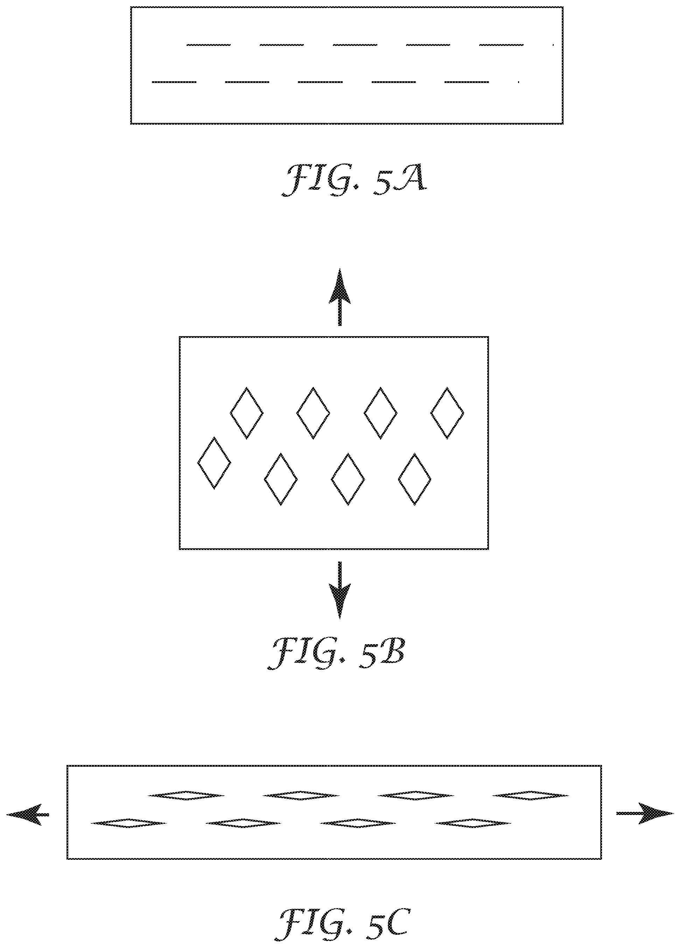

Figures 5A-5C show a scheme for activating and de-activating articles of this disclosure. Figure 5A shows a non-activated article as described above. In Figure 5B the article is stretched in the direction or directions of the arrows. As shown, the stretching causes the slits to form gaps. In Figure 5C, the article in Figure 5B is de-activated by stretching in an orthogonal direction as indicated by the arrows. As shown, the de-activation causes a diminishment in the gaps in the backing layer and may also cause wrinkling in the backing layer to decrease the adhesion of the article.

### EXAMPLES

These examples are merely for illustrative purposes only and are not meant to be limiting on the scope of the appended claims. The following abbreviations are used: cm = centimeters.

### Example 1

A strip of tape (3M Vinyl Tape 471, 3M Company, St. Paul, MN) about 6 cm wide and 12 cm in length was slit lengthwise in a skip-slit pattern. The slits were about 4 cm long and separated by about 1 cm. Rows of slits were also separated by about 1 cm. After slitting, the tape was adhered to a vertical surface with the slits oriented horizontally. A test specimen (3M EASY TRAP Sweep & Dust Sheet, 3M Company, St. Paul, MN) was pressed against the backing of the slitted tape and found not to adhere.

The tape was then removed from the surface and stretched, increasing the width of the tape by about 2 cm and opening the slits. The tape was re-applied to the vertical surface as before. Visual inspection of the tape showed that adhesive was exposed through the backing toward the ends of the slits. The test specimen was again pressed against the backing of the slitted and stretched tape and found to adhere.

## Claims

1. An adhesive article (100; 200; 300; 400) comprising:
a layer of adhesive (120; 320; 420) with a first major surface and a second major surface; and
a first backing layer (110; 210; 410) with a first major surface and a second major surface, wherein the first major surface of the first backing layer (110; 210; 410) is in contact with the second major surface of the adhesive layer (120; 320; 420), and wherein the first backing layer (110; 210; 410) comprises a plurality of cuts, wherein the plurality of cuts are gaps but the gaps are not visible to the naked eye when the adhesive article is in an unstressed state, but in a stressed state, at least some of the cuts become gaps that permit adhesion of the second major surface of the adhesive layer (120; 320; 420) to a substrate surface; wherein the stressed state comprises stretching, bending or a combination thereof.

2. The adhesive article of claim 1, wherein the plurality of cuts is arrayed in a pattern along one axis or more than one axis.

3. The adhesive article of claim 1, wherein at least some of the plurality of cuts are continuous cuts .

4. The adhesive article of claim 1, wherein the adhesive comprises a gel adhesive or a pressure sensitive adhesive.

5. The adhesive article of claim 1, wherein the adhesive layer (120; 320; 420) comprises a multi-layer adhesive, wherein the multi-layer adhesive comprises at least 2 adhesive layers (120; 320; 420), a first adhesive layer comprising the first major surface of the adhesive layer (120; 320; 420) and a second adhesive layer comprising the second major surface of the adhesive layer (120; 320; 420), and wherein the first and second adhesive layers (120; 320; 420) are the same or different.

6. The adhesive article of claim 1, wherein the first backing layer (110; 210; 410) comprises a thermoplastic film of polyolefin, polyurethane, polyester, or polyether block amide.

7. The adhesive article (400) of claim 1, further comprising a second backing layer (440) with a first major surface and a second major surface, wherein the second major surface of the second backing layer (440) is in contact with the first major surface of the adhesive layer (120; 320; 420), and wherein the second backing layer (440) comprises a plurality of cuts, wherein the plurality of cuts are gaps but the gaps are not visible to the naked eye when the adhesive article is in an unstressed state, but in a stressed state, at least some of the cuts become gaps that permit adhesion of the layer of adhesive to a substrate surface.

8. An adhesive construction comprising:
a first substrate surface; and
an adhesive article (100; 200; 300; 400) according to claim 1 attached to the first substrate surface, wherein the first major surface of the layer of adhesive is in
contact with the first substrate surface.

9. The adhesive construction of claim 8, wherein the second substrate comprises a wearable medical device, a sensor, a catheter, a vacuum port, a controller for an insulin pump, or a monitor.

10. A method of preparing an adhesive article (100; 200; 300; 400) comprising:
providing a first backing layer (110; 210; 410) with a first major surface and a second major surface;
modifying the first backing layer (110; 210; 410) by cutting a plurality of cuts into the first backing
layer without substantially removing material from the first backing layer (110; 210; 410), such that in
an unstressed state the cuts are gaps, but the gaps are not visible to the naked eye but
in a stressed state at least some of the gaps become visible to the naked eye;
providing an adhesive; and
forming a layer of adhesive on the first major surface of the first backing layer (110; 210; 410) such that the adhesive layer (120; 320; 420) has a first major surface and a second major surface and the second major surface of the adhesive layer (120; 320; 420) is in contact with first major surface of the first backing layer (110; 210; 410).

11. The method of claim 10, wherein modifying the backing layer (110; 210; 410) by cutting a plurality of cuts into the backing layer (110; 210; 410) comprises cutting with a knife, a blade, a water jet, or a laser beam.

12. The method of claim 10, further comprising:
providing a second backing layer (440) with a first major surface and a second major surface, where the second backing layer (440) comprises a plurality of cuts, wherein the cuts
are gaps, but the gaps are not visible to the naked eye but in a stressed state at least some of the gaps become visible to the naked eye; and
contacting the second major surface of the second backing layer (440) to the first major
surface of the adhesive layer (120; 320; 420).

## Patentansprüche

1. Ein Klebeartikel (100; 200; 300; 400), aufweisend:
eine Klebeschicht (120; 320; 420) mit einer ersten Hauptoberfläche und einer zweiten Hauptoberfläche; und
eine erste Trägerschicht (110; 210; 410) mit einer ersten Hauptoberfläche und einer zweiten Hauptoberfläche, wobei die erste Hauptoberfläche der ersten Trägerschicht (110; 210; 410) in Kontakt mit der zweiten Hauptoberfläche der Klebeschicht (120; 320; 420) steht und wobei die erste Trägerschicht (110; 210; 410) eine Mehrzahl von Schnitten aufweist, wobei die Mehrzahl von Schnitten Lücken sind, die Lücken jedoch mit bloßem Auge nicht sichtbar sind, wenn sich der Klebeartikel in einem unbelasteten Zustand befindet, aber in einem belasteten Zustand mindestens einige der Schnitte zu Lücken werden, die ein Kleben der zweiten Hauptoberfläche der Klebeschicht (120; 320; 420) an einer Substratoberfläche ermöglichen; wobei der belastete Zustand ein Dehnen, ein Biegen oder eine Kombination davon aufweist.

2. Der Klebeartikel nach Anspruch 1, wobei die Mehrzahl von Schnitten in einem Muster entlang einer Achse oder mehr als einer Achse angeordnet ist.

3. Der Klebeartikel nach Anspruch 1, wobei mindestens einige der Mehrzahl von Schnitten durchgehende Schnitte sind.

4. Der Klebeartikel nach Anspruch 1, wobei der Klebstoff einen Gelklebstoff oder einen Haftklebstoff aufweist.

5. Der Klebeartikel nach Anspruch 1, wobei die Klebeschicht (120; 320; 420) einen mehrschichtigen Klebstoff aufweist, wobei der mehrschichtige Klebstoff mindestens 2 Klebeschichten (120; 320; 420) aufweist, eine erste Klebeschicht, aufweisend die erste Hauptoberfläche der Klebeschicht (120; 320; 420), und eine zweite Klebeschicht, aufweisend die zweite Hauptoberfläche der Klebeschicht (120; 320; 420), und wobei die erste und die zweite Klebeschicht (120; 320; 420) gleich oder unterschiedlich sind.

6. Der Klebeartikel nach Anspruch 1, wobei die erste Trägerschicht (110; 210; 410) eine thermoplastische Folie aus Polyolefin, Polyurethan, Polyester oder Polyetherblockamid aufweist.

7. Der Klebeartikel (400) nach Anspruch 1, ferner aufweisend eine zweite Trägerschicht (440) mit einer ersten Hauptoberfläche und einer zweiten Hauptoberfläche, wobei die zweite Hauptoberfläche der zweiten Trägerschicht (440) in Kontakt mit der ersten Hauptoberfläche der Klebeschicht (120; 320; 420) steht, und wobei die zweite Trägerschicht (440) eine Mehrzahl von Schnitten aufweist, wobei die Mehrzahl von Schnitten Lücken sind, die Lücken jedoch mit bloßem Auge nicht sichtbar sind, wenn sich der Klebeartikel in einem unbelasteten Zustand befindet, aber in einem belasteten Zustand mindestens einige der Schnitte zu Lücken werden, die ein Kleben der Klebeschicht an einer Substratoberfläche ermöglichen.

8. Eine Klebekonstruktion, aufweisend:
eine erste Substratoberfläche; und
einen Klebeartikel (100; 200; 300; 400) nach Anspruch 1, der an der ersten Substratoberfläche befestigt ist, wobei die erste Hauptoberfläche der Klebeschicht in Kontakt mit der ersten Substratoberfläche steht.

9. Die Klebekonstruktion nach Anspruch 8, wobei das zweite Substrat eine tragbare medizinische Vorrichtung, einen Sensor, einen Katheter, einen Vakuumanschluss, einen Controller für eine Insulinpumpe oder einen Monitor aufweist.

10. Ein Verfahren zum Herstellen eines Klebeartikels (100; 200; 300; 400), aufweisend:
Bereitstellen einer ersten Trägerschicht (110; 210; 410) mit einer ersten Hauptoberfläche und einer zweiten Hauptoberfläche;
Modifizieren der ersten Trägerschicht (110; 210; 410) durch Schneiden einer Mehrzahl von Schnitten in die erste Trägerschicht, ohne wesentliches Entfernen von Material von der ersten Trägerschicht (110; 210; 410), derart, dass
in einem unbelasteten Zustand die Schnitte Lücken sind, aber die Lücken mit bloßem Auge nicht sichtbar sind, aber
in einem belasteten Zustand mindestens einige der Lücken mit bloßem Auge sichtbar werden;
Bereitstellen eines Klebstoffs; und
Ausbilden einer Klebeschicht auf der ersten Hauptoberfläche der ersten Trägerschicht (110; 210; 410), derart, dass die Klebeschicht (120; 320; 420) eine erste Hauptoberfläche und eine zweite Hauptoberfläche hat und die zweite Hauptoberfläche der Klebeschicht (120; 320; 420) mit der ersten Hauptoberfläche der ersten Trägerschicht (110; 210; 410) in Kontakt steht.

11. Das Verfahren nach Anspruch 10, wobei das Modifizieren der Trägerschicht (110; 210; 410) durch das Schneiden einer Mehrzahl von Schnitten in die Trägerschicht (110; 210; 410) das Schneiden mit einem Messer, einer Klinge, einem Wasserstrahl oder einem Laserstrahl aufweist.

12. Das Verfahren nach Anspruch 10, ferner aufweisend:
Bereitstellen einer zweiten Trägerschicht (440) mit einer ersten Hauptoberfläche und einer zweiten Hauptoberfläche, wobei die zweite Trägerschicht (440) eine Mehrzahl von Schnitten aufweist, wobei die Schnitte
Lücken sind, aber die Lücken mit bloßem Auge nicht sichtbar sind, aber in einem belasteten Zustand mindestens
einige der Lücken mit bloßem Auge sichtbar werden; und
Kontaktieren der zweiten Hauptoberfläche der zweiten Trägerschicht (440) mit der ersten Hauptoberfläche
der Klebeschicht (120; 320; 420).

## Revendications

1. Article adhésif (100 ; 200 ; 300 ; 400) comprenant :
une couche d'adhésif (120 ; 320 ; 420) avec une première surface principale et une seconde surface principale ; et
une première couche de support (110 ; 210 ; 410) avec une première surface principale et une seconde surface principale, dans lequel la première surface principale de la première couche de support (110 ; 210 ; 410) est en contact avec la seconde surface principale de la couche adhésive (120 ; 320 ; 420), et dans lequel la première couche de support (110 ; 210 ; 410) comprend une pluralité de découpes, dans lequel la pluralité de découpes sont des espaces, mais les espaces ne sont pas visibles à l'œil nu lorsque l'article adhésif est dans un état non contraint, mais dans un état contraint, au moins certaines des découpes deviennent des espaces qui permettent une adhésion de la seconde surface principale de la couche adhésive (120 ; 320 ; 420) à une surface de substrat ; dans lequel l'état contraint comprend un étirement, un cintrage ou une combinaison de ceux-ci.

2. Article adhésif selon la revendication 1, dans lequel la pluralité de découpes est disposée dans un motif le long d'un axe ou plus d'un axe.

3. Article adhésif selon la revendication 1, dans lequel au moins certaines parmi la pluralité de découpes sont des découpes continues.

4. Article adhésif selon la revendication 1, dans lequel l'adhésif comprend un adhésif en gel ou un adhésif sensible à la pression.

5. Article adhésif selon la revendication 1, dans lequel la couche adhésive (120 ; 320 ; 420) comprend un adhésif multicouche, dans lequel l'adhésif multicouche comprend au moins 2 couches adhésives (120 ; 320 ; 420), une première couche adhésive comprenant la première surface principale de la couche adhésive (120 ; 320 ; 420) et une seconde couche adhésive comprenant la seconde surface principale de la couche adhésive (120 ; 320 ; 420), et dans lequel les première et seconde couches adhésives (120 ; 320 ; 420) sont identiques ou différentes.

6. Article adhésif selon la revendication 1, dans lequel la première couche de support (110 ; 210 ; 410) comprend un film thermoplastique de polyoléfine, de polyuréthane, de polyester, ou de polyéther bloc amide.

7. Article adhésif (400) selon la revendication 1, comprenant en outre une seconde couche de support (440) avec une première surface principale et une seconde surface principale, dans lequel la seconde surface principale de la seconde couche de support (440) est en contact avec la première surface principale de la couche adhésive (120 ; 320 ; 420), et dans lequel la seconde couche de support (440) comprend une pluralité de découpes, dans lequel la pluralité de découpes sont des espaces, mais les espaces ne sont pas visibles à l'œil nu lorsque l'article adhésif est dans un état non contraint, mais dans un état contraint, au moins certaines des découpes deviennent des espaces qui permettent une adhésion de la couche d'adhésif à une surface de substrat.

8. Construction adhésive comprenant :
une première surface de substrat ; et
un article adhésif (100 ; 200 ; 300 ; 400) selon la revendication 1 fixé à la première surface de substrat, dans laquelle la première surface principale de la couche d'adhésif est en contact avec la première surface de substrat.

9. Construction adhésive selon la revendication 8, dans laquelle le second substrat comprend un dispositif médical pouvant être porté, un capteur, un cathéter, un orifice à vide, un organe de commande pour une pompe à insuline, ou un moniteur.

10. Procédé de préparation d'un article adhésif (100 ; 200 ; 300 ; 400) comprenant :
la fourniture d'une première couche de support (110 ; 210 ; 410) avec une première surface principale et une seconde surface principale ;
la modification de la première couche de support (110 ; 210 ; 410) en découpant une pluralité de découpes dans la première couche de support
sans retirer sensiblement de matériau de la première couche de support (110 ; 210 ; 410), de telle sorte que dans
un état non contraint les découpes sont des espaces, mais les espaces ne sont pas visibles à l'œil nu, mais
dans un état contraint au moins certains des espaces deviennent visibles à l'œil nu ;
la fourniture d'un adhésif ; et
la formation d'une couche d'adhésif sur la première surface principale de la première couche de support (110 ; 210 ; 410) de telle sorte que la couche adhésive (120 ; 320 ; 420) a une première surface principale et une seconde surface principale et la seconde surface principale de la couche adhésive (120 ; 320 ; 420) est en contact avec la première surface principale de la première couche de support (110 ; 210 ; 410).

11. Procédé selon la revendication 10, dans lequel la modification de la couche de support (110 ; 210 ; 410) en découpant une pluralité de découpes dans la couche de support (110 ; 210 ; 410) comprend le découpage avec un couteau, une lame, un jet d'eau ou un faisceau laser.

12. Procédé selon la revendication 10, comprenant en outre :
la fourniture d'une seconde couche de support (440) avec une première surface principale et une seconde surface principale, où la seconde couche de support (440) comprend une pluralité de découpes, dans lequel les découpes
sont des espaces, mais les espaces ne sont pas visibles à l'œil nu, mais dans un état contraint au moins
certains des espaces deviennent visibles à l'œil nu ; et
la mise en contact de la seconde surface principale de la seconde couche de support (440) avec la première surface principale
de la couche adhésive (120 ; 320 ; 420).
